# EUROPEAN PATENT APPLICATION

(11) **EP 4 563 198 A2**
(43) Date of publication of application: **04.06.2025**
(21) Application number: 24216618.9
(22) Date of filing: 29.11.2024
(51) Int. Cl.: A61N 5/10, A61B 90/00, A61B 90/14

(54) **FIDUCIAL FRAME AND SUPPORT DEVICE**

(30) Priority: 01.12.2023 GB 202318444
(71) Applicant: Elekta Instrument AB, 103 93 Stockholm (SE)
(72) Inventor: FAHLBORG, Philip, Stockholm (SE); MARKSTRÖM, Mårten, Stockholm (SE)
(74) Representative: Finlayson, Scott Henry

(57) **Abstract**

The present disclosure relates to a fiducial frame for mounting around a patient's head during an x-ray positioning procedure. The fiducial frame comprising: a first radiolucent panel located at a first side of the frame, and a second radiolucent panel located at a second side of the frame, wherein the first panel comprises a first plurality of fiducial positioning markers, and a first identifying marker having a first asymmetric position relative to the first plurality of positioning markers; wherein the second panel comprises a second plurality of fiducial positioning markers, and a second identifying marker having a second asymmetric position relative to the second plurality of positioning markers; wherein the first asymmetric position relative to the first plurality of positioning markers is different from the second asymmetric position relative to the second plurality of positioning markers.

## Description

### Technical Field

The present disclosure relates to a fiducial frame for mounting around a patient's head during an x-ray positioning procedure, and to a support device for such a fiducial frame.

### Background

During stereotactic radiotherapy ("SRT"), fine beams of radiation are delivered to a tumour or otherwise diseased tissue in a patient, from multiple different angular positions around the body of the patient. Each beam is focussed on the diseased tissue, such that the maximal dose of radiation is delivered to the tumour, while only a limited dose of radiation is delivered to surrounding tissue. Because of the high positional accuracy achievable by SRT, it is commonly used for treating diseased tissue in the brain. However, diseased tissue located elsewhere in the body can also be treated with SRT.

In order to maximise the dose delivered to diseased tissue, and minimise the dose delivered to healthy tissue, it is important to prevent movement of the treatment site during treatment. It is also important to know precisely where the diseased tissue is positioned in the reference frame of the SRT machine.

Where SRT is performed on the brain, a stereotactic frame is used to prevent movement of the patient's head. Stereotactic frames attach securely to the patient's head, thereby preventing movement of the patient's head during treatment.

Once the stereotactic frame has been securely attached to the patient's head, it is then important to accurately determine the position of the tumour relative to the stereotactic frame. By accurately determining the position of the diseased tissue relative to the stereotactic frame, and then attaching the stereotactic frame to a clamp having a known position relative to the SRT machine, the radiation beams can be correctly directed towards the treatment site.

It is known to use x-ray imaging to facilitate the locating of diseased tissue relative to a stereotactic frame. In particular, it is known to attach a positioning frame having fiducial positioning markers to the stereotactic frame, and to take x-ray images of the patient's head when attached to the positioning frame. The fiducial positioning markers provide a frame of reference for the x-ray images, such that the exact position of the tumour relative to the stereotactic frame can be accurately determined with the help of treatment planning software.

A problem exists, however, that treatment planning by means of existing x-ray positioning frames can be time-consuming, and is not impervious to error. Accordingly, there exists a need for improved x-ray positioning frames.

### Summary

The inventors have particularly found that confusion between x-ray images captured using existing x-ray positioning frames can lead to the image information being unusable for treatment planning, which slows the treatment planning as the images have to be re-taken. In some cases, there is a risk that confusion between the captured x-ray images may lead to mis-location of the diseased tissue.

According to a first aspect, there is provided a fiducial frame for mounting around a patient's head during an x-ray positioning procedure, the fiducial frame comprising:
a first radiolucent panel located at a first side of the frame, and a second radiolucent panel located at a second side of the frame,
wherein the first panel comprises a first plurality of fiducial positioning markers, and a first identifying marker having a first asymmetric position relative to the first plurality of positioning markers;
wherein the second panel comprises a second plurality of fiducial positioning markers, and a second identifying marker having a second asymmetric position relative to the second plurality of positioning markers;
wherein the first asymmetric position relative to the first plurality of positioning markers is different from the second asymmetric position relative to the second plurality of positioning markers.

By providing a fiducial frame in which the two radiolucent panels have differently positioned asymmetric identifying markers with respect to one another, the two radiolucent panels can be easily distinguished from one another. In particular, when an x-ray image shows an identifying marker having the first asymmetric position, it is known that the x-ray image pertains to the first panel; and when an x-ray image shows an identifying marker having the second asymmetric position, it is known that the x-ray image pertains to the second panel. Accordingly, confusion between x-ray images is avoided. In other words, the identifying markers provide for quick and easy identification of x-ray images.

The x-ray positioning procedure may comprise taking x-ray images the patient's head with the fiducial frame mounted around the patient's head. The images may be processed using treatment planning software. The fiducial positioning markers may define a fixed and predefined frame of reference for the x-ray images.

The fiducial positioning markers and the identifying markers may be radiopaque, such that they clearly show up in the x-ray images.

Each panel may comprise a flat sheet of radiolucent material. The first panel may be for positioning at the front of the patient's head. It may have a cut-out to accommodate the patient's nose. The second panel may be for positioning at a side of the patient's head.

The frame may take the form of a box having a first side, a second side, a third side, and a fourth side. The first and third sides may be parallel to one another. The second and fourth sides may be parallel to one another and perpendicular to the first and third sides. The first panel may be located on the first side of the box, and the second panel may be located on the second side of the box.

Each identifying marker may be located so as not to be equidistant between any two fiducial positioning markers.

The first plurality of fiducial positioning markers may be arranged in a first grid (e.g. first symmetric grid). The second plurality of fiducial positioning markers may be arranged in a second grid (e.g. second symmetric grid). The first and second symmetric grids may be identical to one another. That is to say, if the first and second panels were to be placed over one another, the two grids may perfectly align with one another. The first identifying marker may be arranged asymmetrically with respect to the first symmetric grid. The second identifying marker may be arranged asymmetrically with respect to the second symmetric grid.

For example, where each symmetric grid comprises a square or rectangular grid of four fiducial positioning markers, each of the first and second identifying markers may be located asymmetrically with respect to the square or rectangular grid, e.g. located so as not to be equidistant between any two fiducial positioning markers.

In other examples, each symmetric grid may comprise three fiducial positioning markers, e.g. arranged at three corners of a symmetric grid.

The first position may be a first distance from a respective one of the first plurality of fiducial positioning markers, and the second position may be a second distance from a corresponding one of the second plurality of fiducial positioning markers, wherein the first distance is greater than the second distance.

The fiducial frame may further comprise a third radiolucent panel, and a fourth radiolucent panel. Each of the third and the fourth radiolucent panels may comprise a respective plurality of fiducial positioning markers. The third and fourth radiolucent panels may be perpendicular to one another. The third panel may be parallel to the first panel, on an opposite side of the frame from the first panel. The fourth panel may be parallel to the second panel, on an opposite side of the frame from the second panel. Where the fiducial frame takes the form of a box having a first side, a second side, a third side, and a fourth side, the third panel may be located at the third side, and the fourth panel may be located at the fourth side.

The third and fourth panels may be free from asymmetric identifying markers.

Each fiducial positioning marker on the first radiolucent panel and each fiducial positioning marker on the second radiolucent panel may be a first type of fiducial marker. Each fiducial positioning marker on the third radiolucent panel and each fiducial positioning marker on the fourth radiolucent panel may be a second type of fiducial marker. The first type being visually distinct from the second type. For example, the first type of fiducial marker may be a "plus"-type fiducial marker, and the second type of fiducial marker may be a "cross"-type fiducial marker.

At least one of the radiolucent panels may comprises a first alignment line extending in a first direction and/or a second alignment line extending in a second direction which is perpendicular to the first direction, the first alignment line being spaced from the second alignment line. The alignment lines may be visible to the naked eye. They may be radiolucent. The alignment lines may be used to align an x-ray camera with respect to the panels, e.g. by eye.

In one example, the alignment lines may comprise openings in the panels. For example, the panels may have a colour tint, for example a grey colour tint or a blue colour tint, such that the openings are clearly visible.

The fiducial frame may further comprise means for attachment to a stereotactic frame. For example, the fiducial frame may further comprise a support structure for attachment to a stereotactic frame, wherein the radiolucent panels are attached to the support structure. The support structure may comprise a box-like structure to which the panels are attached. The support structure may comprise attachment portions for attachment to a stereotactic frame at a predefined position and orientation relative to the stereotactic frame.

The support frame may be formed of radiolucent material. The stereotactic frame may be formed of radiolucent material.

Herein, a stereotactic frame is defined as a frame for attachment directly to a patient's head. Attachment may be by screws which engage the patient's head, so as to prevent movement of the frame relative to the patient's head.

In a second aspect there is provided a stereotactic apparatus comprising a stereotactic frame, and a fiducial frame according to the first aspect configured for attachment thereto. The fiducial frame may be configured for attachment to the stereotactic frame a predetermined fixed position relative thereto.

In a third aspect there is provided an x-ray positioning apparatus comprising a fiducial frame according to the first aspect or a stereotactic apparatus according to the second aspect, and a support device for attachment to the fiducial frame or stereotactic apparatus, wherein the support device comprises a radiolucent material. The support device may be configured for attachment to or mounting on a patient support surface, for example a patient bed.

By using a radiolucent material, the fiducial frame does not obscure x-ray images captured of the apparatus, and in particular does not obscure any of the positioning markers.

In a fourth aspect there is provided a support device for securing a fiducial frame to a patient support surface, the support device comprising attachment means for attachment to the fiducial frame, and a clamp formed of radiolucent material, wherein the clamp is configured for attachment to a patient support surface, and wherein the attachment means is mounted to the clamp.

The attachment means may be means for attachment to the fiducial frame according to the first aspect, or for attachment to a stereotactic frame, for example a stereotactic frame according to the third aspect. The attachment means may comprise a bracket configured to attach to an attachment surface on the fiducial frame or stereotactic frame.

The radiolucent material may comprise plastic or carbon fiber-reinforced plastic.

In a fifth aspect there is provided a support device for mounting a fiducial frame on a patient support surface, the support device comprising:
a sheet of radiolucent material for placement on a patient support surface; and
attachment means mounted to the sheet of radiolucent material, the attachment means configured for attachment to the fiducial frame.

The attachment means may be means for attachment to the fiducial frame according to the first aspect, or for attachment to a stereotactic frame, for example a stereotactic frame according to the third aspect. The attachment means may comprise a bracket configured to attach to an attachment surface on the fiducial frame or stereotactic frame.

The radiolucent material may comprise plastic or carbon fiber-reinforced plastic.
the sheet of radiolucent material is configured for placement under a patient's head, or wherein the sheet of radiolucent material is configured for placement under a patient's torso. An underside of the sheet comprises an anti-slip material.

In a sixth aspect there is provided a system comprising:
a fiducial frame according to the first aspect or a stereotactic apparatus according to the second aspect; and
a support device according to the third, fourth, or fifth aspects.

### Brief Description of the Drawings

Embodiments of the present disclosure will now be described, with reference to the accompanying drawings, in which:
Figure 1A shows a perspective view of a fiducial frame 100;
Figure 1B shows a front view of the fiducial frame 100;
Figure 1C shows a first lateral view of the fiducial frame 100;
Figure 1D shows a back view of the fiducial frame 100;
Figure 1E shows a second lateral view of the fiducial frame 100;
Figure 1F shows a top view of a fiducial frame 100;
Figure 1G shows a bottom view of a fiducial frame 100;
Figure 2A shows a first x-ray image of the fiducial frame 100 mounted on a patient's head;
Figure 2B shows a second x-ray image of the fiducial frame 100 mounted on a patient's head;
Figures 3A-3B depict a support device 300 for securing a fiducial frame to a patient support surface;
Figures 4A-5B depict a support device 400 for securing a fiducial frame to a patient support surface; and
Figure 6 depicts a fiducial frame 100 attached to a stereotactic frame 600.

Like reference numerals are used for like components throughout the drawings.

### Detailed Description

As noted in the Summary section above, the inventors have found that confusion between x-ray images captured using existing x-ray positioning frames can lead to the image information being unusable for treatment planning, which slows the treatment planning as the images have to be re-taken. In some cases, there is a risk that confusion between the captured x-ray images may lead to mis-location of the diseased tissue.

The claimed invention provides a fiducial frame in which two radiolucent panels have differently positioned asymmetric identifying markers with respect to one another, allowing for the two radiolucent panels to be easily distinguished from one another. In particular, when an x-ray image shows an identifying marker having the first asymmetric position, it is known that the x-ray image pertains to the first panel; and when an x-ray image shows an identifying marker having the second asymmetric position, it is known that the x-ray image pertains to the second panel. Accordingly, confusion between x-ray images is avoided.

Such a fiducial frame can be mounted around a patient's head during an x-ray positioning procedure, in order to provide a frame of reference for x-ray images, such that the position of, for example, a tumour relative to a stereotactic frame attached to the patient's head may be determined with the help of treatment planning software. Typically, 2D images acquired during an x-ray positioning procedure are arranged, or registered, into a 3D stack of images using treatment planning software. Any confusion between x-ray images can risk the 2D images being arranged, or registered, inaccurately. Providing the software with x-ray images in which radiolucent panels of the fiducial frame are easily distinguishable from each other thus allows for a more accurate and reliable arrangement of 2D images into such a 3D stack.

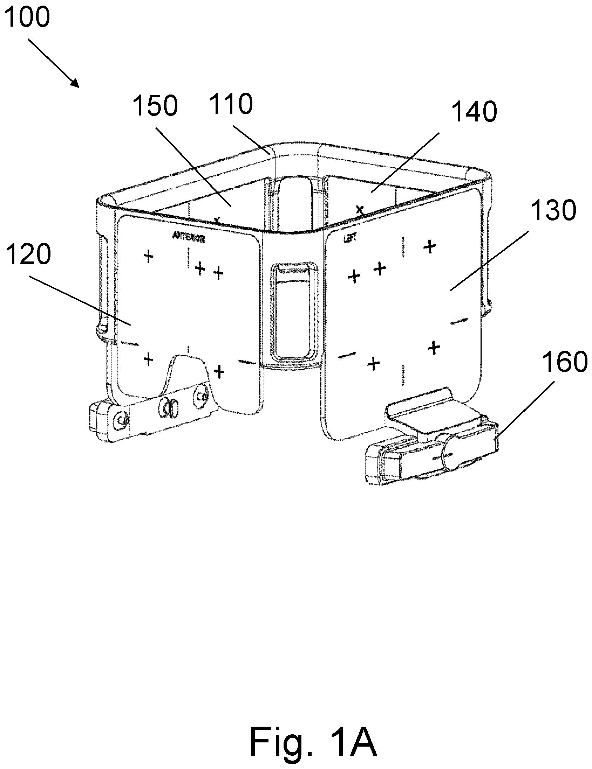

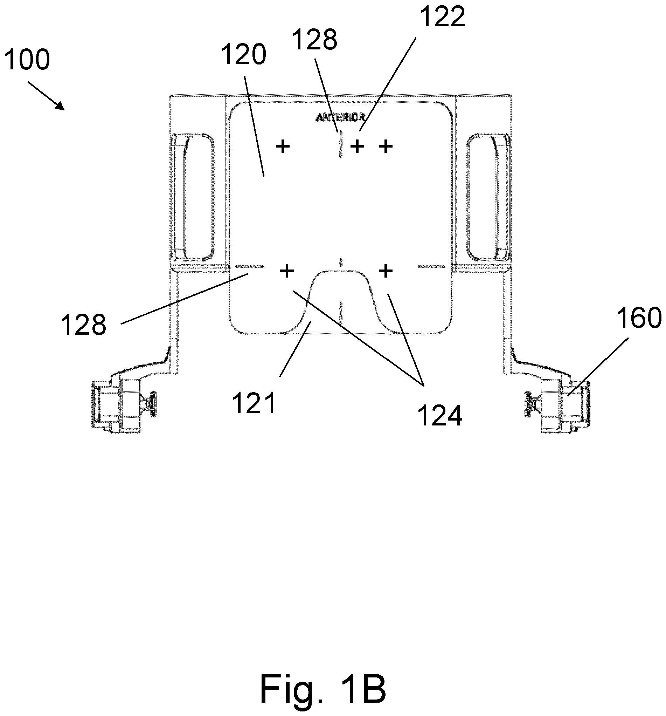

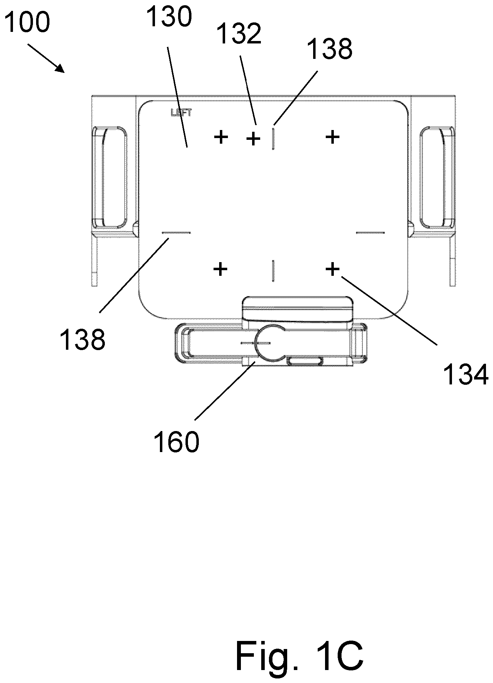

Figure 1A shows a perspective view of a fiducial frame 100 according to the claimed invention. The fiducial frame 100 comprises a first radiolucent panel 120 located at a first side of the frame, and a second radiolucent panel 130 located at a second side of the frame. Each of the first radiolucent panel 120 and second radiolucent panel 130 comprises a plurality of markers. The markers are described in further detail with respect to figure 1B and figure 1C. In some implementations, the fiducial frame further comprises a third radiolucent panel 140 located at a third side of the fiducial frame, and a fourth radiolucent panel 150 located at a fourth side of the frame.

The first radiolucent panel 120 may be arranged perpendicularly to the second radiolucent panel 120, as depicted in figure 1A. In other words, the first side of the fiducial frame may be perpendicular to the second side of the frame. Similarly, the third radiolucent panel 140 may be arranged perpendicularly to the fourth radiolucent panel 150, as depicted in figure 1A. In further implementations, the third radiolucent panel 140 can be arranged parallel to the first radiolucent panel 120, on an opposite side of the fiducial frame from the first radiolucent panel 120. Similarly, the fourth radiolucent panel 150 can be arranged parallel to the second radiolucent panel 130 on an opposite side of the fiducial frame from the second radiolucent panel 130. In other words, the first, second, third and fourth radiolucent panels may be arranged such as to form a fiducial box.

The fiducial frame 100 may comprise a support structure 110 for attachment to a stereotactic frame as shown, for example, in figure 1A. The first radiolucent panel 120 and second radiolucent panel 130 are attached to the support structure 110. In some implementations, the third radiolucent panel140 and the fourth radiolucent panel 150 are also attached to the support structure 110. Such a support structure 110 may be formed of radiolucent material. In some implementations, the support structure 110 may be formed of radiolucent material comprising plastic or carbon fiber-reinforced plastic.

The use of radiolucent material in the formation of the support structure is advantageous, as radiation (such as x-rays) can pass through the radiolucent material of the support structure relatively unobstructed. In other words, the use of radiolucent material in the formation of the support structure allows the support structure to be almost entirely invisible in x-ray images. A radiolucent material may therefore refer to a material which is substantially permeable to radiation, for example x-ray radiation. By contrast, a radiopaque material may refer to a material which is substantially impermeable to radiation, for example x-ray radiation.

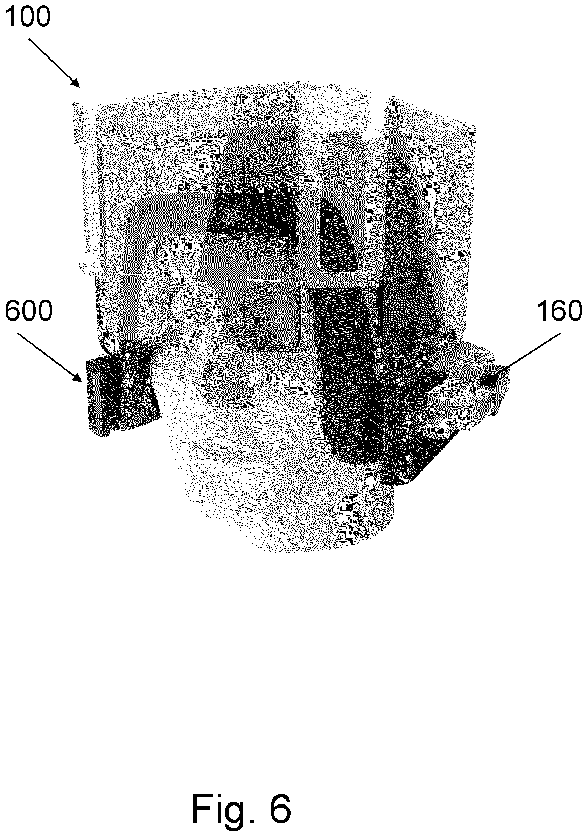

The fiducial frame 100 can also comprise means for attachment 160 to a stereotactic frame (shown in more detail in figure 6). Such means for attachment 160 may comprise more than one attachment component, and may include, for example, a clamp mechanism, a slot mechanism, or a mechanical attachment such as screws or the like. The means for attachment 160 may be attached to, fixed to, or form part of, the second radiolucent panel 130, as shown in figure 1A. Optionally, the means for attachment may be attached to, fixed to, or form part of the second radiolucent panel 130 and the fourth radiolucent panel 150, as shown in figure 1A. In other examples, the means for attachment 160 may be attached to, fixed to, or form part of the support structure 110.

Figure 1B depicts a front view, otherwise known as an anterior view, of the fiducial frame 100. The first radiolucent panel 120 comprises a first plurality of fiducial positioning markers 124. Fiducial positioning markers 124 are markers used to provide a frame of reference for imaging purposes - for example, fiducial positioning markers may be objects or marks placed in the field of view of an imaging device or system, which then appear in the resulting images. In the present invention, fiducial positioning markers on the radiolucent panels of the fiducial frame 100 are placed such as to be in the field of view of an x-ray imaging system during an x-ray positioning procedure. In this way, the fiducial positioning markers provide a frame of reference for the x-ray images acquired during the x-ray positioning procedure. Accordingly, because the position of the fiducial frame relative to the stereotactic is known (c.f. figure 6), the position of the frame of reference relative to the stereotactic frame is known. Accordingly, by processing the x-ray images based on the frame of reference provided by the positioning markers on the fiducial frame, the position of the diseased tissue relative to the stereotactic frame can be calculated.

The plurality of fiducial positioning markers 124 can include three or more fiducial positioning markers. In some implementations, the plurality of fiducial positioning markers comprises at least four fiducial positioning markers arranged in a grid. An exemplary arrangement of the fiducial positioning markers 124 may be seen in figure 1A and figure 1B, but it is noted that these figures are merely exemplary, and that a number of different arrangements may be used within the scope of the invention. Nonetheless, using four fiducial positioning markers as shown in figure 1B may be preferred in some systems, as the four fiducial positioning markers arranged in a grid provide a cartesian frame of reference which may simplify treatment planning.

The fiducial positioning markers 124 can be radiopaque, to enable to fiducial positioning markers to be visible on x-ray images.

The first radiolucent panel further comprises a first identifying marker 122. As shown, the first identifying marker 122 is asymmetrically arranged relative to the symmetrically arranged 2x2 grid of fiducial positioning markers 124. In particular, the first identifying marker 122 has a first asymmetric position relative to the first plurality of positioning markers. An example of this asymmetric positioning is shown in figure 1A and figure 1B, but it is noted that these figures are merely exemplary, and that a number of different asymmetric positionings may be used within the scope of the invention. The asymmetric position of the first identifying marker 122 may be a first distance from a respective one of the first plurality of fiducial positioning markers 124.

The first identifying marker 122 can be radiopaque, to enable the first identifying marker to be visible on x-ray images.

Similar to the fiducial positioning markers 124, the first identifying marker 122 provides a reference for imaging purposes, as the first identifying marker 122 acts as a differentiator between x-ray images pertaining to different panels of the fiducial frame 100. In particular, when an x-ray image of the fiducial frame shows an identifying marker 122 having the first asymmetric position, it is known that the x-ray image pertains to the first radiolucent panel 120.

The first radiolucent panel 120 may also be referred to as the front panel or the anterior view panel. As shown, it includes a cutaway section for accommodating the patient's nose 121. This cutaway may enable the panel to be closer to the patient's face, thus increasing measurement accuracy.

Optionally, the first radiolucent panel 120 can comprise a first alignment line 128 extending in a first direction and/or a second alignment line 128 extending in a second direction which is perpendicular to the first direction, the first alignment line being spaced from the second alignment line. The first alignment line and/or second alignment line may partially or fully extend across the first radiolucent panel 120 in the respective first and/or second direction. The first alignment line 128 may extend in a vertical direction, and the second alignment line 128 may extend in a horizontal direction (as defined when the patient is wearing the frame 100 and is standing up), or vice versa. The first direction may therefore be parallel to the sagittal plane of the patient, and the second direction may be parallel to the transverse plane of the patient. In other implementations, the first alignment line and/or second alignment line may extend diagonally across the first radiolucent panel 120. Each of the first alignment line and/or the second alignment line may comprise a continuous line. A dashed line, a dotted line, or a segmented line, may however alternatively be used. In some examples, the alignment lines 128 may be formed as openings through the panel 120, particularly where the panel 120 has a colour tint. Preferably, the alignment lines are visible to the naked eye.

Advantageously, the first alignment line and/or second alignment line allow for the fiducial frame 100 to be aligned appropriately with respect to an imaging system such as an x-ray imaging system. In other words, the presence of the alignment line or lines on the fiducial frame allows for any misalignment of the fiducial frame or of the x-ray camera to be perceived, for example by a clinician, prior to the x-ray positioning procedure. Such misalignment may be with respect to the imaging system, the patient support surface, etc. Once perceived by the clinician, the misalignment can be corrected prior to the implementation of x-ray positioning procedure. Optionally, the first alignment line and or second alignment line may be radiolucent, so that the alignment lines do not obscure x-ray images acquired during the x-ray positioning procedure.

Figure 1C depicts a first lateral view of the fiducial frame 100. As shown, the second radiolucent panel 130 comprises a second plurality of fiducial positioning markers 134. Similar to the first radiolucent panel 120, the plurality of fiducial positioning markers 134 can include three or more fiducial positioning markers. In some implementations, the plurality of fiducial positioning markers comprises at least four fiducial positioning markers arranged in a grid. An exemplary arrangement of the fiducial positioning markers 134 may be seen in figure 1C, but it is noted that these figures are merely exemplary, and that a number of different arrangements may be used within the scope of the invention. The layout of and spacing between the fiducial positioning markers 134 on the second panel 130 may be identical to that of the fiducial positioning markers 124 on the first panel 120. According, the frame of reference provided by the first panel 120 may have the same scale and proportions as the frame of reference provided by the second panel 130.

The fiducial positioning markers 134 can be radiopaque, to enable to fiducial positioning markers to be visible on x-ray images.

The second radiolucent panel 130 further comprises a second identifying marker 132, where the second identifying marker 132 has a second asymmetric position relative to the second plurality of positioning markers 134. An example of this asymmetric positioning is shown in figure 1C, but it is noted that these figures are merely exemplary, and that a number of different asymmetric positionings may be used within the scope of the invention. The second asymmetric position of the second identifying marker 132 relative to the second plurality of positioning markers 134 is different to the first asymmetric position of the first identifying marker 122 relative to the first plurality of positioning markers 124. In other words, the first identifying marker 122 and the second identifying marker 132 are in different positions on their respective panels. The first identifying marker 122 is in a first asymmetric position on the first panel 120, for example as shown in figure 1B, and the second identifying marker 132 is in a second asymmetric position on the second panel 130, for example as shown in figure 1C. Advantageously, the differently positioned asymmetric identifying markers allow the first radiolucent panel and the second radiolucent panel to be easily distinguished from one another. For example, when an x-ray image of the fiducial frame 100 shows an identifying marker having the first asymmetric position, it is known that the x-ray image pertains to the first panel 120; and when an x-ray image of the fiducial frame 100 shows an identifying marker having the second asymmetric position, it is known that the x-ray image pertains to the second panel 130. Accordingly, confusion between x-ray images of the fiducial frame 100 is avoided.

The asymmetric position of the second identifying marker 132 may be a second distance from a respective one of the second plurality of fiducial positioning markers 134, where the first distance between the first asymmetric position and first plurality of positioning markers 124 is greater than this second distance. In the same or other implementations, the asymmetric position of the first identifying marker 122 on the first radiolucent panel 120 may be at a first angle relative to a respective one of the first plurality of fiducial positioning markers 124, where the asymmetric position of the second identifying marker 132 on the second radiolucent panel 130 may be at a second angle relative to a respective one of the second plurality of fiducial positioning markers 134, the first angle being different to the second angle.

The second identifying marker 132 can be radiopaque, to enable the first identifying marker to be visible on x-ray images.

Similar to the fiducial positioning markers 134, the first identifying marker 132 provides a reference for imaging purposes, as the first identifying marker 132 acts as a differentiator between x-ray images pertaining to different panels of the fiducial frame 100. In particular, when an x-ray image of the fiducial frame shows an identifying marker 132 having the second asymmetric position, it is known that the x-ray image pertains to the second radiolucent panel 130.

The second radiolucent panel 130 may also be referred to as the left-side panel or the first lateral view panel.

The second radiolucent panel 130 may comprise a first alignment line 138 and/or second alignment line 138, where the alignment lines are the same as or similar to those described with respect to the first radiolucent panel 120.

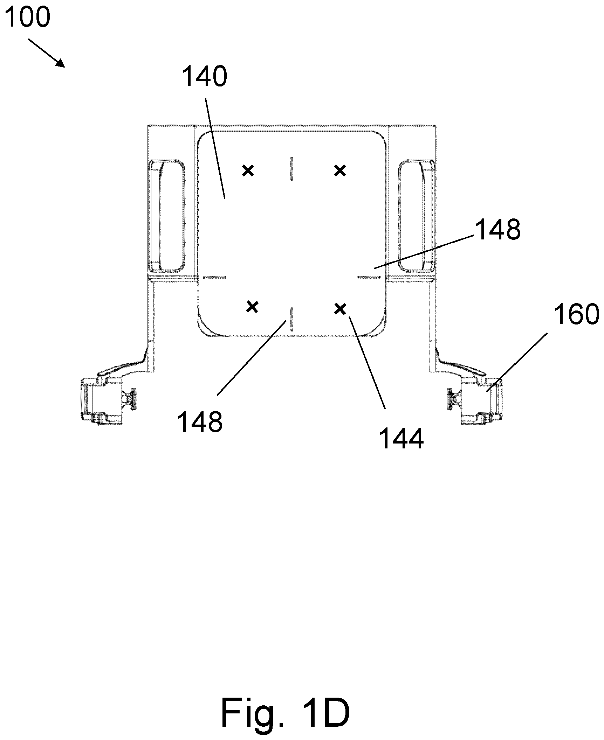

In some implementations, the fiducial frame 100 further comprises a third radiolucent panel 140, as shown in figure 1D. The third radiolucent panel comprises a plurality of fiducial markers, which may be arranged similarly or the same as the plurality of fiducial positioning markers 124 of the first radiolucent panel 120. In other words, the third radiolucent panel can comprise a third plurality of fiducial markers 144, where the plurality can comprise three or more fiducial markers, or may include at least four fiducial markers arranged in a grid.

Each fiducial positioning marker on the first radiolucent panel and each fiducial positioning marker on the second radiolucent panel are a first type of fiducial marker. In contrast, each fiducial marker of the third plurality of fiducial markers is a second type of fiducial marker, the second type of fiducial marker being visually distinct from the first type of fiducial marker. For example, as shown in figures 1A to 1C, the first type of fiducial positioning marker may be a "plus"-type fiducial marker, and the second type of fiducial marker may be a "cross"-type fiducial marker. The visual distinction between the first type of fiducial marker and the second type of fiducial marker allow for the panels to be distinguished from one another. In particular, the distinction between the types of fiducial markers allows for treatment planning software to distinguish between x-ray images of the panels. As will be described further below, x-ray images acquired of, for example, the first panel 120, can be distinguished from those acquired of the third panel 140, due to the visual distinction between the respective markers on the first panel 120 and third panel 140.

The third radiolucent panel 140 may also be referred to as the back panel or the posterior view panel.

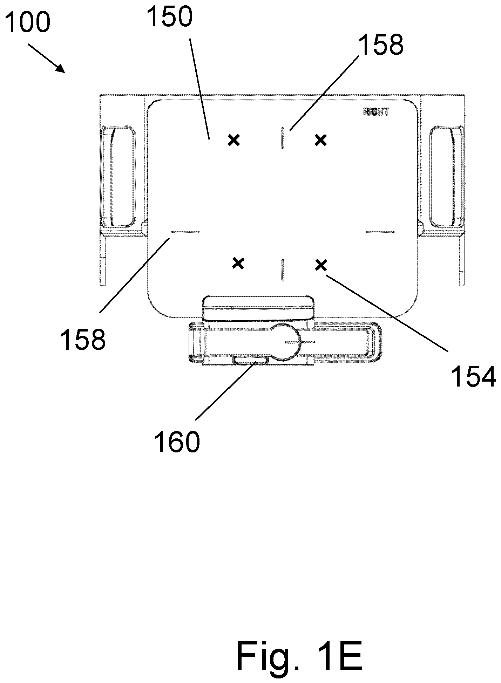

In such implementations, the fiducial frame 100 further comprises a fourth radiolucent panel 150, as shown in figure 1E. The fourth radiolucent panel comprises a plurality of fiducial markers, which may be arranged similarly or the same as the plurality of fiducial positioning markers 134 of the second radiolucent panel 130. In other words, the fourth radiolucent panel can comprise a fourth plurality of fiducial markers 154, where the plurality can comprise three or more fiducial markers, or may include at least four fiducial markers arranged in a grid. Each marker of the fourth plurality of fiducial markers is a fiducial marker of the second type of fiducial markers. As described with respect to the third radiolucent panel 140, the distinction between the types of fiducial markers allows for treatment planning software to distinguish between x-ray images of the panels. As will be described further below, x-ray images acquired of, for example, the second panel 130, can be distinguished from those acquired of the fourth panel 150, due to the visual distinction between the respective markers on the second panel 130 and fourth panel 150.

The fourth radiolucent panel 150 may also be referred to as the right-side panel or the second lateral view panel.

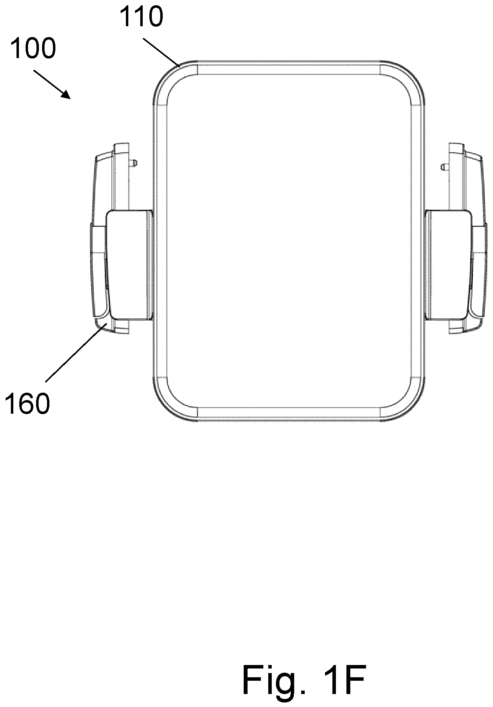

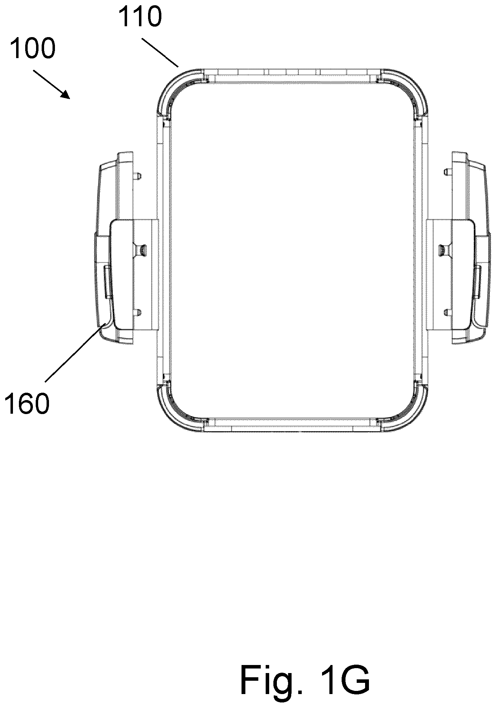

Figure 1F shows a top view of the fiducial frame 100, and figure 1G shows a bottom view of the fiducial frame 100. As can be seen, the frame has a rectangular shape when viewed in this direction. It therefore resembles a box. As can be seen from figure 1F, no panel is present at the top of the frame 100. However, in some examples, a panel may also be provided at the top of the frame 100. The panel may have fiducial positioning markers, similarly to the panels described above. The fiducial positioning markers may have a third type which is visually distinct from the first and second types.

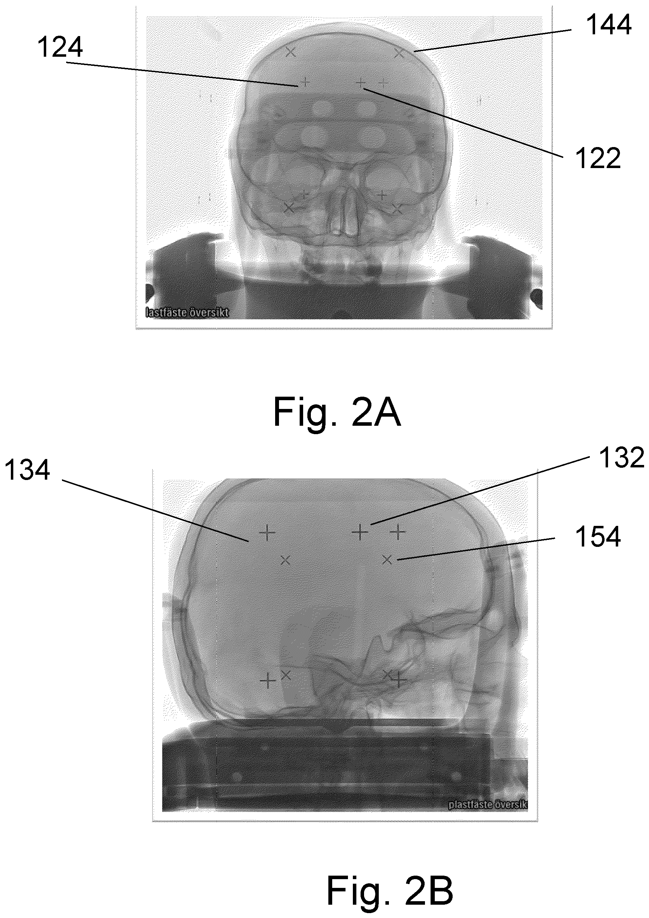

Figure 2A shows an x-ray image of the fiducial frame 100 mounted on a patient's head during an x-ray positioning procedure. The first plurality of fiducial positioning markers 124 are visible on the x-ray image, along with the first identifying marker 122. The third plurality of fiducial positioning markers 144 are also visible on the image, and appear smaller and closer together than the first plurality 124 as the first and second panels are located on opposite sides of the patient's head from one another. Due to the relative placement of the first plurality of positioning markers 124 and the third plurality of positioning markers 144, the perspective view pertaining to the image can be easily determined. Furthermore, the view is known to be a front to back view because the first positioning marker 122 (as opposed to the second positioning marker 132) can be seen.

Figure 2B shows an different x-ray image of the fiducial frame 100 mounted on a patient's head during an x-ray positioning procedure. The second plurality of fiducial positioning markers 134 are visible on the x-ray image, along with the second identifying marker 132. The fourth plurality of fiducial positioning markers 154 are also visible on the image, and appear smaller and closer together than the second plurality of markers 134 as the second and fourth panels are on opposite sides of the patient's head from one another. Due to the relative placement of the second plurality of positioning markers 134 and the fourth plurality of positioning markers 154, the perspective view pertaining to the image can be easily determined. Furthermore, the view is known to be a side to side view because the second positioning marker 132 (as opposed to the first positioning marker 122) can be seen.

In short, as can be seen from Figures 2A and 2B, the presence of the identifying markers 122/132, and the use of different types of positioning markers, enables the perspective pertaining to each image to be easily determined with confidence.

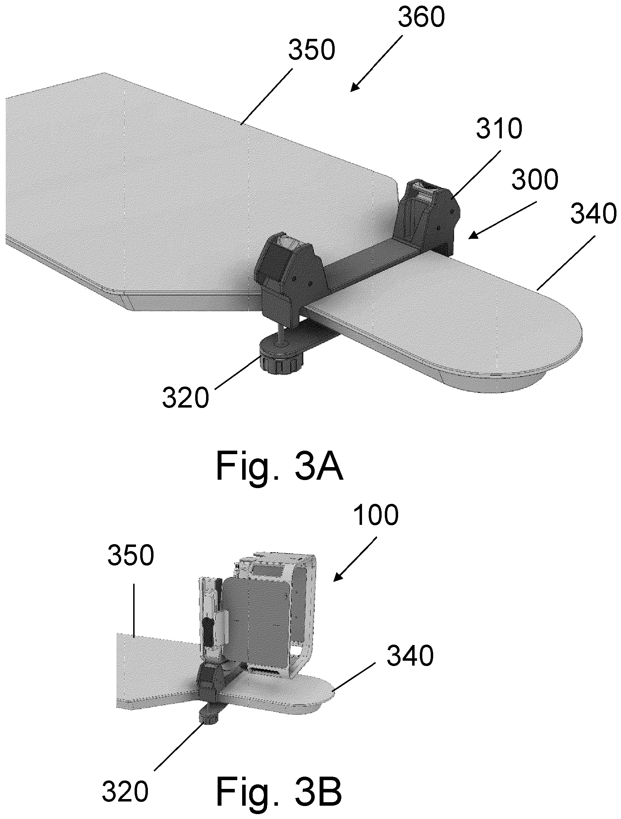

Figure 3A depicts a support device 300 for securing a fiducial frame to a patient support surface 360, e.g. patient bed or x-ray table. For example, support device 300 can be used to secure fiducial frame 100 to a patient support surface 360, as shown in figure 3B. The support device 300 comprises attachment means 310 for attachment to the fiducial frame (or, in some examples, for attachment to the stereotactic frame to which the fiducial frame is attached), and a clamp 320 formed of radiolucent material. The clamp 320 is configured for attachment to the patient support surface 360, and the attachment means 310 is mounted to the clamp 320, for example as shown in figure 3A. The attachment means 310 can comprise any suitable arrangement for attaching or fixing a fiducial frame to a patient support surface. The attachment means 310 may work in tandem with the attachment means 160 on the fiducial frame 100 to secure the fiducial frame 100 to the patient support surface 360 (or, in some examples, for attachment to the stereotactic frame to which the fiducial frame is attached). In some implementations, the attachment means 310 may comprise a slot into which the attachment means 160 of the fiducial frame is fixed (or, in some examples, for attachment to the stereotactic frame to which the fiducial frame is attached). In other implementations, the attachment means can comprise mechanical attachment means, such as screws, bolts and the like.

The use of radiolucent material in the formation of the clamp is advantageous, as radiation (such as x-rays) can pass through the radiolucent material of the clamp unobstructed. In other words, the use of radiolucent material in the formation of the clamp allows the clamp to be almost entirely invisible in x-ray images. In this way, the clamp does not interfere with any x-ray images of the fiducial frame taken during an x-ray positioning procedure, allowing for more accurate and complete images to be acquired. The radiolucent material can comprise, for example, plastic or carbon fiber-reinforced plastic, both of which are radiolucent.

The patient support surface 360 may not be a uniformly shaped or dimensioned table, and instead can comprise a wider portion 350, suitable for a patient torso, and a narrower portion 340, suitable for a patient head. The support device 300 can be clamped to the patient support surface 350 at the juncture between the wider portion and narrower portion of the patient support surface, as shown in figure 3A.

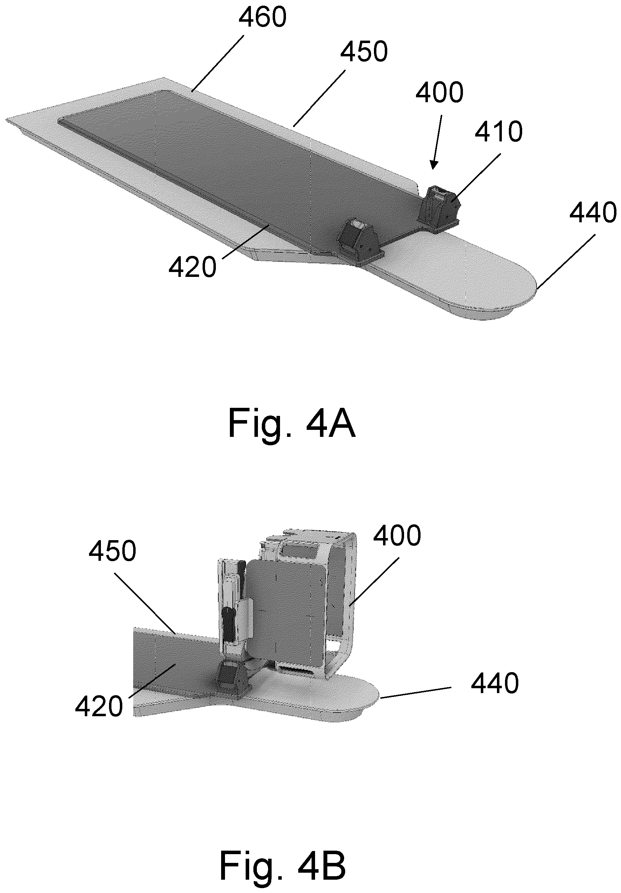

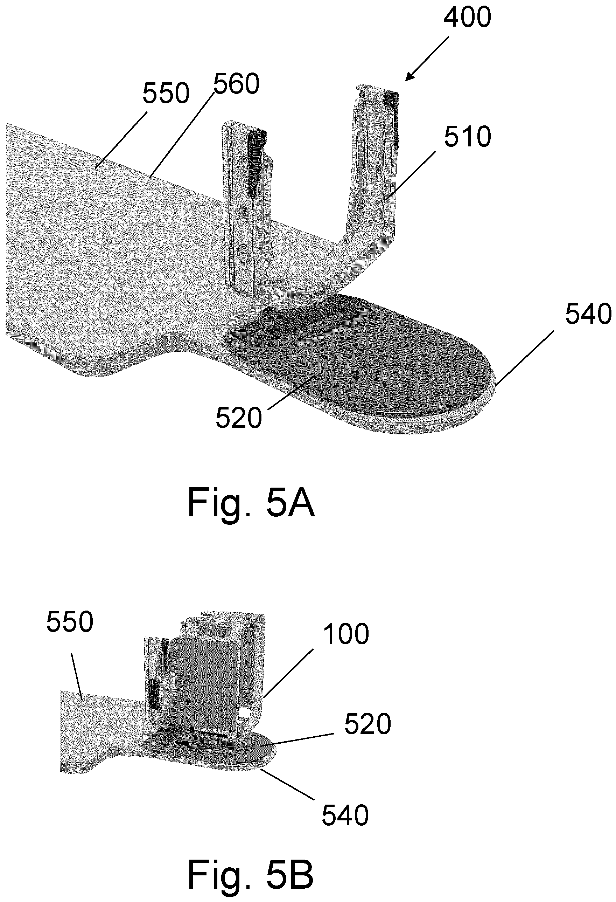

Figure 4A and 5A depict embodiments of a support device 400 for mounting a fiducial frame on a patient support surface 460. The support device 400 comprises a sheet of radiolucent material 420 for placement on the patient support surface 460. Attachment means 410 are mounted to the sheet of radiolucent material, the attachment means 410 configured for attachment to the fiducial frame (or, in some examples, for attachment to the stereotactic frame to which the fiducial frame is attached). The attachment means 410 may be similar to, or the same as, the attachment means 310 described with respect to support device 300.

As with support device 300, the attachment means 410/510 are mounted to the sheet of radiolucent material such that when the support device is placed on the patient support surface 460 that the attachment means are positioned to receive the fiducial frame (or, in some examples, for attachment to the stereotactic frame to which the fiducial frame is attached). For example, for patient support surface 460/560, the attachment means are mounted to the sheet of radiolucent material such that the attachment means are at the juncture between the wider portion 450/550 and narrower portion 440/540 of the patient support surface 460/560.

The use of a sheet of radiolucent material is advantageous for a number of reasons, as radiation (such as x-rays) can pass through the radiolucent material of the sheet unobstructed. In other words, the use of radiolucent material allows the sheet to be almost entirely invisible in x-ray images. In this way, the sheet does not interfere with any x-ray images of the fiducial frame taken during an x-ray positioning procedure, allowing for more accurate and complete images to be acquired. The radiolucent material can comprise, for example, plastic or carbon fiber-reinforced plastic.

As the sheet 420 is for placement on the patient support surface 460, the weight of a patient on the patient support surface 460 can be utilised to fix and/or promote stability of the fiducial frame, for example during an x-ray positioning procedure. The sheet 420 may be configured for placement under a patient's torso in a first embodiment, as shown in figure 4A, or may be configured for placement under a patient's head in a second embodiment, as shown in figure 5A. The configuration of support device 400 allows for a fiducial frame to be mounted to a patient support surface while reducing the amount of material present around a patient's head, thus providing a support device which is lighter and easier to use than those present in the art. In addition, reducing the amount of material present around a patient's head during an x-ray positioning procedure can reduce the amount of distortion present in any acquired x-ray images.

In more detail, figure 4A shows the first embodiment of support device 400, wherein the sheet of radiolucent material 420 is configured for placement under the patient's torso. The sheet 420 is shown as bring uniform and rectangular in shape, but it is noted that this figure is merely exemplary, and many other configurations of the sheet 420 are possible. For example, the sheet 420 may be dimensioned so as to cover the entirety of the wider portion of the patient support surface 460, or may be a curved or a non-uniform shape. Figure 4B depicts the fiducial frame 100 attached to the patient support surface 460 via the support device 400.

As noted above, the weight of a patient on the sheet of radiolucent material 460, and thus on the patient support surface 460, can be utilised to fix and/or promote stability of the fiducial frame, for example during an x-ray positioning procedure. Looking at figure 4A, it can be seen that the weight of the patient torso can act on the sheet of radiolucent material 560 when a patient is present on the patient positioning surface 460, fixing the support device 400 to the patient support surface, and promoting stability.

In more detail, figure 5A shows the second embodiment of support device 400, wherein the sheet of radiolucent material 520 is configured for placement under the patient's head. The sheet 520 is shown as bring uniform and rectangular in shape, but it is noted that this figure is merely exemplary, and many other configurations of the sheet 520 are possible. For example, the sheet 520 may be dimension so as to cover the entirety of the narrower portion of the patient support surface 560, or may be a curved or a non-uniform shape. The attachment means 510 in this embodiment also comprises a frame holder 510, as shown in figure 5A. The frame holder 510 can be dimensioned such as to securely fit a fiducial frame, such as fiducial frame 100, within the holder, allowing the fiducial frame to be fixed in place with respect to the patient support surface. The fiducial frame (or, in some examples, the stereotactic frame to which the fiducial frame is attached) may slide or slot into place within the frame holder 510. In other words, the frame holder 510 is configured to receive the fiducial frame (or, in some examples, the stereotactic frame to which the fiducial frame is attached), and may further comprise one or more slots for receiving the fiducial frame/stereotactic frame.

As noted above, the weight of a patient on the sheet of radiolucent material 520, and thus on the patient support surface 560, can be utilised to fix and/or promote stability of the fiducial frame, for example during an x-ray positioning procedure. Looking at figure 5B, which depicts the fiducial frame 100 attached to the patient support surface 560 via the support device 500, it can be seen that the weight of the patient head and fiducial frame 100 acts on the sheet of radiolucent material 520, fixing the support device 400 to the patient support surface, and promoting stability.

In some implementations, the underside of the sheet material of support device 400 may comprise an anti-slip material.

Figure 6 depicts the fiducial frame 100 attached to a stereotactic frame 600. As noted, a stereotactic frame is used to prevent movement of the patient's head during stereotactic radiotherapy ("SRT"). Stereotactic frames attach securely to the patient's head, thereby preventing movement of the patient's head during treatment. As shown in figure 6, attachment means 160 may also be utilised to attach or fix the fiducial frame 100 to the stereotactic frame 600. The arrangement shown in Figure 6 may correspond to the second aspect.

It is to be understood that the above description is intended to be illustrative, and not restrictive. Many other implementations will be apparent to those of skill in the art upon reading and understanding the above description. Although the present disclosure has been described with reference to specific example implementations, it will be recognized that the disclosure is not limited to the implementations described, but can be practiced with modification and alteration within the spirit and scope of the appended claims. Accordingly, the specification and drawings are to be regarded in an illustrative sense rather than a restrictive sense. The scope of the disclosure should, therefore, be determined with reference to the appended claims, along with the full scope of equivalents to which such claims are entitled.

Also disclosed herein are a number of examples according to the following numbered clauses.
1. A fiducial frame for mounting around a patient's head during an x-ray positioning procedure, the fiducial frame comprising:
   a first radiolucent panel located at a first side of the frame, and a second radiolucent panel located at a second side of the frame,
   wherein the first panel comprises a first plurality of fiducial positioning markers, and a first identifying marker having a first asymmetric position relative to the first plurality of positioning markers;
   wherein the second panel comprises a second plurality of fiducial positioning markers, and a second identifying marker having a second asymmetric position relative to the second plurality of positioning markers;
   wherein the first asymmetric position relative to the first plurality of positioning markers is different from the second asymmetric position relative to the second plurality of positioning markers.
2. The fiducial frame of clause 1, wherein the first radiolucent panel is arranged perpendicularly to the second radiolucent panel.
3. The fiducial frame of clause 1 or clause 2, further comprising means for attachment to a stereotactic frame.
4. The fiducial frame of any preceding clause, wherein the first position is a first distance from a respective one of the first plurality of fiducial positioning markers, and wherein the second position is a second distance from a corresponding one of the second plurality of fiducial positioning markers, wherein the first distance is greater than the second distance.
5. The fiducial frame of any preceding clause, wherein each of the first plurality of fiducial positioning markers and the second plurality of fiducial positioning markers comprises at least three fiducial positioning markers.
6. The fiducial frame of any preceding clause, wherein each of the first plurality of fiducial positioning markers and the second plurality of positioning markers comprises at least four fiducial positioning markers arranged in a grid.
7. The fiducial frame of any preceding clause, further comprising a third radiolucent panel and a fourth radiolucent panel, each comprising a plurality of fiducial positioning markers, the third radiolucent panel being perpendicular to the fourth radiolucent panel.
8. The fiducial frame of clause 7, wherein the third radiolucent panel is arranged parallel to the first radiolucent panel, on an opposite side of the fiducial frame from the first radiolucent panel, and wherein the fourth radiolucent panel is arranged parallel to the second radiolucent panel on an opposite side of the fiducial frame from the second radiolucent panel.
9. The fiducial frame of any preceding clause, wherein each fiducial positioning marker on the first radiolucent panel and each fiducial positioning marker on the second radiolucent panel is a first type of fiducial marker, and wherein each fiducial positioning marker on the third radiolucent panel and each fiducial positioning marker on the fourth radiolucent panel is a second type of fiducial marker, the first type being visually distinct from the second type.
10. The fiducial frame of clause 8 or clause 9, wherein the first type of fiducial marker is a "plus"-type fiducial marker, and the second type of fiducial marker is a "cross"-type fiducial marker.
11. The fiducial frame of any preceding clause, wherein the fiducial positioning markers are radiopaque.
12. The fiducial frame of any preceding clause, wherein at least one of the radiolucent panels comprises a first alignment line extending in a first direction and/or a second alignment line extending in a second direction which is perpendicular to the first direction, the first alignment line being spaced from the second alignment line.
13. The fiducial frame of clause 12, wherein the alignment lines are visible to the naked eye, and optionally are radiolucent.
14. The fiducial frame of any preceding clause, further comprising a support structure for attachment to a stereotactic frame, wherein the radiolucent panels are attached to the support structure.
15. A support device for securing a fiducial frame to a patient support surface, the support device comprising attachment means for attachment to the fiducial frame, and a clamp formed of radiolucent material, wherein the clamp is configured for attachment to a patient support surface, and wherein the attachment means is mounted to the clamp.
16. The support device of clause 15, wherein the radiolucent material comprises plastic or carbon fiber-reinforced plastic.
17. The support device of clause 15 or clause 16, wherein the fiducial frame is a fiducial frame according to any of clauses 1 to 14.
18. A support device for mounting a fiducial frame on a patient support surface, the support device comprising:
   a sheet of radiolucent material for placement on a patient support surface; and
   attachment means mounted to the sheet of radiolucent material, the attachment means configured for attachment to the fiducial frame.
19. The support device of clause 18, wherein the radiolucent material comprises plastic or carbon fiber-reinforced plastic.
20. The support device of clause 18 or clause 19, wherein the fiducial frame is a fiducial frame according to any of any of clauses 1 to 14.
21. The support device according to any of clauses 18 to 20, wherein the sheet of radiolucent material is configured for placement under a patient's head, or wherein the sheet of radiolucent material is configured for placement under a patient's torso.
22. The support device according to any of clauses 18 to 21, wherein an underside of the sheet comprises an anti-slip material.
23. A system comprising:
   a fiducial frame according to any of clauses 1 to 14; and
   a support device according to any of clauses 15 to 22.

## Claims

1. A fiducial frame for mounting around a patient's head during an x-ray positioning procedure, the fiducial frame comprising:
a first radiolucent panel located at a first side of the frame, and a second radiolucent panel located at a second side of the frame,
wherein the first panel comprises a first plurality of fiducial positioning markers, and a first identifying marker having a first asymmetric position relative to the first plurality of positioning markers;
wherein the second panel comprises a second plurality of fiducial positioning markers, and a second identifying marker having a second asymmetric position relative to the second plurality of positioning markers;
wherein the first asymmetric position relative to the first plurality of positioning markers is different from the second asymmetric position relative to the second plurality of positioning markers.

2. The fiducial frame of claim 1, wherein the first radiolucent panel is arranged perpendicularly to the second radiolucent panel.

3. The fiducial frame of claim 1 or claim 2, further comprising means for attachment to a stereotactic frame.

4. The fiducial frame of any preceding claim, wherein the first position is a first distance from a respective one of the first plurality of fiducial positioning markers, and wherein the second position is a second distance from a corresponding one of the second plurality of fiducial positioning markers, wherein the first distance is greater than the second distance.

5. The fiducial frame of any preceding claim, wherein each of the first plurality of fiducial positioning markers and the second plurality of fiducial positioning markers comprises at least three fiducial positioning markers;
and/or wherein each of the first plurality of fiducial positioning markers and the second plurality of positioning markers comprises at least four fiducial positioning markers arranged in a grid.

6. The fiducial frame of any preceding claim, further comprising a third radiolucent panel and a fourth radiolucent panel, each comprising a plurality of fiducial positioning markers, the third radiolucent panel being perpendicular to the fourth radiolucent panel;
optionally wherein the third radiolucent panel is arranged parallel to the first radiolucent panel, on an opposite side of the fiducial frame from the first radiolucent panel, and wherein the fourth radiolucent panel is arranged parallel to the second radiolucent panel on an opposite side of the fiducial frame from the second radiolucent panel.

7. The fiducial frame of any preceding claim, wherein each fiducial positioning marker on the first radiolucent panel and each fiducial positioning marker on the second radiolucent panel is a first type of fiducial marker, and wherein each fiducial positioning marker on the third radiolucent panel and each fiducial positioning marker on the fourth radiolucent panel is a second type of fiducial marker, the first type being visually distinct from the second type.

8. The fiducial frame of claim 6 or claim 7, wherein the first type of fiducial marker is a "plus"-type fiducial marker, and the second type of fiducial marker is a "cross"-type fiducial marker.

9. The fiducial frame of any preceding claim, wherein the fiducial positioning markers are radiopaque.

10. The fiducial frame of any preceding claim, wherein at least one of the radiolucent panels comprises a first alignment line extending in a first direction and/or a second alignment line extending in a second direction which is perpendicular to the first direction, the first alignment line being spaced from the second alignment line;
optionally wherein the alignment lines are visible to the naked eye, and optionally are radiolucent.

11. The fiducial frame of any preceding claim, further comprising a support structure for attachment to a stereotactic frame, wherein the radiolucent panels are attached to the support structure.

12. A support device for securing a fiducial frame to a patient support surface, the support device comprising attachment means for attachment to the fiducial frame, and a clamp formed of radiolucent material, wherein the clamp is configured for attachment to a patient support surface, and wherein the attachment means is mounted to the clamp.

13. The support device of claim 12, wherein the radiolucent material comprises plastic or carbon fiber-reinforced plastic;
and/or wherein the fiducial frame is a fiducial frame according to any of claims 1 to 11.

14. A support device for mounting a fiducial frame on a patient support surface, the support device comprising:
a sheet of radiolucent material for placement on a patient support surface; and
attachment means mounted to the sheet of radiolucent material, the attachment means configured for attachment to the fiducial frame;
optionally wherein the radiolucent material comprises plastic or carbon fiber-reinforced plastic.

15. The support device of claim 14, wherein the fiducial frame is a fiducial frame according to any of any of claims 1 to 11.
